# FASCICULE DE BREVET EUROPEEN

(11) **EP 2 626 075 B1**
(45) Date de publication et mention de la délivrance du brevet: **04.03.2015**
(21) Numéro de dépôt: 13165106.9
(22) Date de dépôt: 08.08.2012
(51) Int. Cl.: A61K 33/00, A61P 23/00, A61K 33/24, A61K 45/06

(54) **Utilisation de xénon inhalé pour prévenir ou traiter des douleurs neuropathiques induites par les sels de platine**
Verwendung von inhaliertem Xenon-Gas zur Vorbeugung oder Behandlung von neuropathischen Schmerzen, die durch Platinsalzen erzeugt werden
Use of inhaled xenon for preventing or treating neuropathic pain induced by platinum salts

(30) Priorité: 14.10.2011 FR 1159296
(43) Date de publication de la demande: 14.08.2013
(62) Demande divisionnaire de: 12179606.4
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BESSIERE, Baptiste, 92130 ISSY LES MOULINEAUX (FR); PYPE, Jan, 1540 HERNE (BE); SIMONNET, Guy, 33200 BORDEAUX (FR)
(74) Mandataire: Pittis, Olivier

(56) Documents cités:
- FR-A1- 2 944 969
- US-A1- 2010 203 156
- MERCADANTE S ET AL: "Breakthrough pain in cancer patients: Pathophysiology and treatment", CANCER TREATMENT REVIEWS 199812 GB, vol. 24, no. 6, décembre 1998 (1998-12), pages 425-432, XP008151643, ISSN: 0305-7372
- BINHAS ET AL: "Douleurs cancereuses par exces de nociception chez l'adulte: mise au point sur les recommandations concernant les traitements antalgiques medicamenteux", ANNALES FRANCAISES D'ANESTHESIE ET DE REANIMATION, MASSON, PARIS, FR, vol. 26, no. 6, 1 juin 2007 (2007-06-01), pages 502-515, XP022121490, ISSN: 0750-7658, DOI: 10.1016/J.ANNFAR.2007.03.029
- WIJAYASIRI L ET AL: "Hereditary neuropathy with liability to pressure palsies and anaesthesia: Peri-operative nerve injury", ANAESTHESIA 200610 GB LNKD- DOI:10.1111/J.1365-2044.2006.04786.X, vol. 61, no. 10, octobre 2006 (2006-10), pages 1004-1006, XP008151637, ISSN: 0003-2409
- HAGEN N A ET AL: "Assessment and management of breakthrough pain in cancer patients: Current approaches and emerging research", CURRENT PAIN AND HEADACHE REPORTS 2008 GB LNKD- DOI:10.1007/S11916-008-0042-1, vol. 12, no. 4, 2008, pages 241-248, XP008151655, ISSN: 1531-3433
- WEIMANN J: "Toxicity of nitrous oxide", BEST PRACTICE AND RESEARCH IN CLINICAL ANAESTHESIOLOGY 200303 GB LNKD- DOI:10.1053/BEAN.2002.0264, vol. 17, no. 1, mars 2003 (2003-03), pages 47-61, XP008151639, ISSN: 1521-6896

## Description

L'invention concerne l'utilisation d'une composition ou d'un médicament inhalable gazeux à base de xénon pour prévenir, i.e. empêcher la survenance, ou pour traiter, i.e. guérir totalement ou partiellement, ou pour au moins minimiser, une douleur neuropathique induite par une substance de chimiothérapie anticancéreuse administrée à un patient atteint d'un cancer.

Le développement de chimiothérapies pour traiter les cancers a conduit à une amélioration notable de la survie des patients et souvent à un retour à une vie normale ou quasi-normale.

Cependant, une chimiothérapie anticancéreuse est généralement neurotoxique et induit souvent des complications importantes.

Plus précisément, les effets secondaires neurotoxiques affectent le système nerveux périphérique en ciblant le corps cellulaire des neurones, l'axone, la gaine de myéline et/ou les cellules gliales. Il en résulte habituellement une neuropathie périphérique induite par la chimiothérapie, comme rappelé par C. Sioka et al., Central and peripheral nervous system toxicity of common chemotherapeutic agents ; Cancer Chemother. Pharmacol.; 2009; 63; p. 761-767).

Plus généralement, la prévalence des neuropathies sensorielles chimio-induites varier de 10% à 80% selon le médicament.

Les agents chimiothérapiques anticancéreux ou substances de chimiothérapies anticancéreuses provoquant ce type de neurotoxicité sont notamment les sels de platine, tel que cisplatine, l'oxaliplatine et le carboplatine ; les taxanes, tel que paclitaxel et docétaxel ; les alcaloïdes, en particulier vincristine, thalidomide et bortezomib.

La douleur neuropathique est dès lors un important effet secondaire pour de nombreux patients qui peut se produire en début de traitement mais aussi comme conséquence tardive du traitement et conduire alors à des douleurs chroniques.

Ainsi, le document S. Couraud et al. ; Hypersensibilité aux sels de platine ; Revue de Pneumo. Clinique ; Vol. 64, n°1 ; p. 20-26 ; février 2008, rappelle que les réactions d'hypersensibilité aux sels de platine sont fréquentes et parfois graves. Ces effets peuvent survenir après 4 à 8 cycles de traitement. Par ailleurs, un intervalle long entre deux lignes de traitement accroît le risque de réaction. La prise en charge médicale doit alors consister, dans les cas simples, en une réintroduction prudente du principe actif, c'est-à-dire du sel de platine utilisé, selon un protocole de désensibilisation ou, dans les cas sévères, à une substitution prudente du sels employés par un autre sel, quand cela est possible.

Dans tous les cas, on comprend que ces réactions d'hypersensibilité aux sels de platine sont un réel problème et ne doivent pas être prises à la légère.

Parfois, la neuropathie peut évoluer même après une seule application de drogue, notamment dans le cas d'un traitement par oxaliplatine, comme montré par M. Stengel et al.; Oxaliplatin-induced painful neuropathy - Flicker of hopé or hopeless pain?; Pain 2009; 144 ; p. 225-226).

De manière analogue, les taxanes sont connus pour conduire à des effets secondaires indésirables, notamment des neuropathies se traduisant par des sensations d'engourdissement ou des picotements, principalement sur les mains et les pieds chez les patients.

Or, jusqu'à présent, aucun médicament ou produit réellement efficace pour prévenir et/ou pour guérir une douleur neuropathique induite par un traitement de chimiothérapie anticancéreuse n'a été proposé.

Par ailleurs, le document FR-A-2944969 propose d'utiliser du protoxyde d'azote à une teneur comprise entre 5 et 45% en volume pour traiter les douleurs chroniques chez l'homme, notamment celles d'origines dysfonctionnelle, inflammatoire, neuropathique ou iatrogénique, notamment les douleurs neuropathiques choisies parmi les douleurs post-zostériennes, diabétiques, centrales, post-accident vasculaire cérébrale, liées à la sclérose en plaques, liés aux traumatismes médullaires, liées au Sida ou au cancer, liées aux chimiothérapies ou d'origine post-opératoire.

Le document US 2010 203156 divulgue l'utilisation du xénon pour traiter la douleur par inhalation.

Toutefois, ce document est totalement silencieux quant à celles résultant d'un traitement par un ou des sels de platine, taxanes ou une autre substance analogue du type alcaloïdes, thalidomide ou bortezomib.

Le problème qui se pose est dès lors de pouvoir disposer d'un médicament efficace apte à prévenir et/ou à guérir, totalement ou partiellement (i.e. atténuer), une (ou des) douleur neuropathique induite par un traitement par chimiothérapie anticancéreuse auquel est soumis un patient, typiquement un patient humain, en particulier un traitement par administration d'une substance contenant un ou plusieurs sels de platine, tel que cisplatine, l'oxaliplatine et le carboplatine, plus particulièrement l'oxaliplatine.

La solution selon l'invention porte sur un médicament gazeux inhalable contenant du xénon (Xe) en tant que principe actif pour une utilisation par inhalation pour prévenir et/ou pour traiter au moins une douleur neuropathique induite par au moins une substance de chimiothérapie anticancéreuse administrée à un patient atteint d'un cancer, ladite substance de chimiothérapie anticancéreuse contenant un ou plusieurs composés choisis parmi les sels de platine.

Dans le cadre de l'invention, on précise que :
- par « prévenir », on entend empêcher la survenance ;
- par « traiter », on entend guérir totalement ou partiellement, y compris diminuer, minimiser ou réduire ;
- par « patient », on entend un individu atteint d'un cancer ;
- par « inhalable », on entend administrable par inhalation ; et
- par « % », on entend un pourcentage volumique, i.e. % en volume, sauf indication différente.

Selon le cas, le médicament inhalable de l'invention, c'est-à-dire la composition médicamenteuse gazeuse de l'invention, peut comprendre l'une ou plusieurs des caractéristiques techniques suivantes :
- la substance de chimiothérapie anticancéreuse contient un composé choisi parmi les sels de platine.
- la substance de chimiothérapie anticancéreuse contient plusieurs composés choisis parmi les sels de platine.
- la substance de chimiothérapie anticancéreuse est choisie parmi les sels de platine, tel que cisplatine, l'oxaliplatine, le carboplatine ;
- la substance de chimiothérapie anticancéreuse contient un ou plusieurs sels de platine choisis parmi la cisplatine, l'oxaliplatine et le carboplatine.
- la substance de chimiothérapie anticancéreuse contient de l'oxaliplatine qui est couramment utilisée dans le traitement des cancers du colon ou du rectum, en particulier comme le cancer du colon au stade III (stade C de Dukes) et le traitement des cancers colorectaux métastatiques.
- le patient est atteint d'un cancer du colon ou du rectum.
- le patient est atteint d'un cancer du sein, du poumon (non à petites cellules), de l'ovaire ou de la prostate ou d'un cancer gastrique ou des voies aérodigestives supérieures.
- la proportion volumique efficace de xénon est comprise entre 5 et 70%, préférentiellement moins de 60%.
- la proportion volumique efficace de xénon est inférieure ou égale à 50%, voire inférieure à 40%.
- il contient une proportion volumique efficace de xénon comprise entre 10 et 50%.
- il contient une proportion volumique efficace de xénon comprise entre 10 et 30%.
- il contient en outre une proportion volumique d'oxygène (O₂) d'au moins 21%, typiquement entre 21% et 50%.
- il contient en outre du protoxyde d'azote (N₂O).
- il est administré par inhalation pendant au moins 15 minutes par jour, de préférence au moins 30 min/jour, de préférence encore au moins 1 h/jour.
- il est administré par inhalation pendant une durée suffisante pour obtenir un effet observable dans le ou les jours suivant son administration par inhalation.
- il est administré par inhalation de manière continue ou fractionnée, c'est-à-dire à plusieurs reprises ou en plusieurs fois.
- il peut-être co-administré avec la substance de chimiothérapie anticancéreuse, à savoir notamment une substance de chimiothérapie anticancéreuse contenant un ou plusieurs composés choisis parmi les sels de platine.
- il est administré par inhalation pendant au moins toute la période de temps de traitement du patient par la substance de chimiothérapie anticancéreuse. Ainsi, si le patient doit prendre la substance de chimiothérapie anticancéreuse pendant 6 mois par exemple, alors le médicament est administré également pendant cette même durée de 6 mois, voire un peu plus longtemps pour pallier à des éventuels effets retards.
- il contient, en outre, un gaz additionnel choisi parmi l'argon, l'hélium, le néon, le krypton, le NO, le CO, le sulfure d'hydrogène (H₂S) et l'azote, y compris de l'air.
- il est co-administré avec la substance de chimiothérapie anticancéreuse, c'est-à-dire avant, pendant et/ou après l'administration de la substance de chimiothérapie anticancéreuse.
- le patient est un être humain, c'est-à-dire un homme ou une femme, y compris les enfants, les adolescents ou tout autre groupe d'individus, par exemple les nouveau-nés ou les personnes âgées.
- il est prêt à l'emploi.
- il est conditionné dans une bouteille de gaz.

En d'autres termes, selon la présente invention, on propose d'inhaler du xénon afin de prévenir ou de traiter de façon durable, c'est-à-dire pendant un ou des jours, semaines ou mois, les douleurs neuropathiques susceptibles d'être induites par une (ou plusieurs) substance de chimiothérapie anticancéreuse, c'est-à-dire un agent anticancéreux, choisie parmi les sels de platine, en particulier induites par une substance telle que l'oxaliplatine.

L'invention repose donc sur une co-administration de la substance chimiothérapique anticancéreuse choisie parmi les sels de platine, et du mélange gazeux comprenant du xénon afin de prévenir ou traiter les douleurs neuropathiques chimio-induites.

La concentration et/ou la durée d'administration la plus adaptée à un patient donné peut être choisie(s) de manière empirique par le personnel soignant, par exemple en fonction de l'état de santé ou physique du patient, de la sévérité de la douleur, de son sexe, de son âge, du type de cancer, du type d'agent anticancéreux co-administré, etc...

Le mélange gazeux ou médicament de l'invention peut être mis en oeuvre dans le cadre d'une méthode de traitement thérapeutique, dans laquelle on administre le mélange gazeux par inhalation, par exemple au moyen d'un masque respiratoire soit directement relié à une source de xénon à la concentration requise, par exemple une bouteille de gaz prêt à l'emploi, ou alors à la sortie d'un mélangeur de gaz alimenté par plusieurs sources de gaz (O₂, Xe...) de manière à obtenir le mélange souhaité ; soit relié à un ventilateur respiratoire alimenté en le ou les gaz souhaités.

Dit autrement, la présente invention est donc basée sur l'utilisation d'un mélange gazeux thérapeutique contenant du xénon en tant que produit actif en une proportion efficace pour fabriquer un médicament inhalable à effets curatifs ou préventifs, destiné à traiter les douleurs neuropathiques induites par une substance utilisée dans le cadre d'une chimiothérapie anticancéreuse et administrée à un patient atteint d'un cancer, ladite substance étant choisie parmi les sels de platine, en particulier une substance du type de l'oxaliplatine.

Le xénon sont donc, selon l'invention, mis en oeuvre dans le cadre d'une méthode de traitement thérapeutique, dans laquelle le xénon est administré par inhalation à un mammifère, en particulier un patient humain, pour prévenir ou traiter les douleurs neuropathiques induites par une substance anticancéreuse utilisée en tant que chimiothérapie anticancéreuse administrée audit patient.

La présente invention va maintenant être mieux comprise grâce à la description suivante et aux exemples qui vont suivre, qui sont donnés à titre purement illustratif et en références aux Figures annexées parmi lesquelles :
- la Figure 1 représente un schéma de la molécule d'oxaliplatine ; et
- la Figure 2 représente les effets du N₂O (hors invention) et du xénon (invention) sur la douleur neuropathique induite par l'oxaliplatine chez le rat.

### Exemples

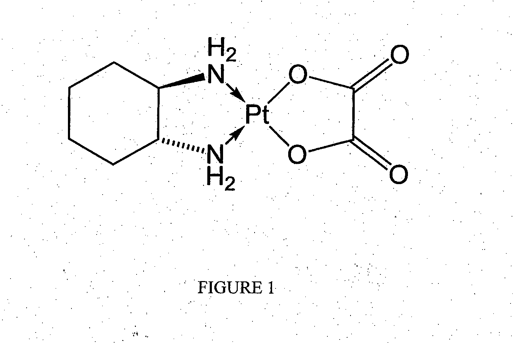

La Figure 1 représente un schéma de la molécule d'oxaliplatine qui a été utilisée dans les essais ci-après comme substance anti-cancéreuse.

L'oxaliplatine est couramment utilisée pour lutter contre les cancers, notamment du rectum et du colon, car elle permet une inhibition de la synthèse et de la réplication de l'ADN par formation de ponts intra-brins entre des guanines adjacentes, ce qui est un traitement de choix dans la lutte contre ce type de cancers. Cette molécule présente malheureusement l'inconvénient d'engendrer des douleurs neuropathiques chez les patients cancéreux auxquels elle est administrée.

De là, afin de démontrer l'efficacité du gaz à base de N₂O (hors invention) ou de xénon selon l'invention dans la lutte contre ce type de douleur neuropathiques induites par l'oxaliplatine, des essais comparatifs ont été réalisés dans les conditions suivantes.

Une étude préclinique chez le rat a été menée.

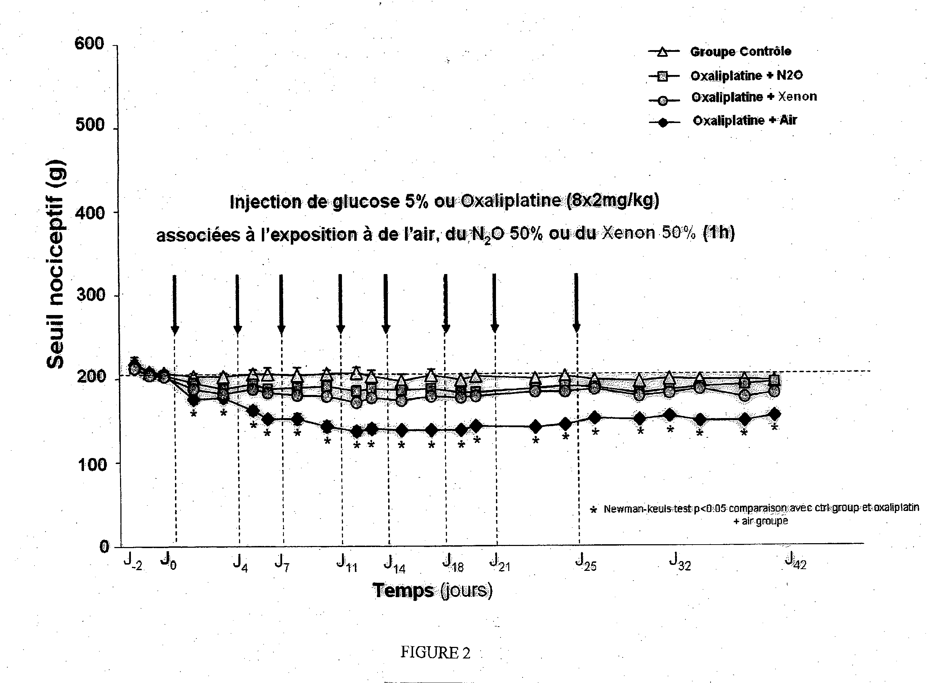

Afin de mimer au mieux l'utilisation clinique, une série de 8 injections intra-péritonéales d'oxaliplatine ont été réalisées tous les 3 à 4 jours comme illustré sur la Figure 2. Ce modèle est bien connu dans la littérature et rend compte des douleurs neuropathiques chimio-induites.

Les seuils de douleurs ont été évalués à l'aide d'un dispositif de Randall-Selitto en appliquant une pression croissante sur la patte du rat jusqu'à l'émission d'un cri par l'animal. Ensuite, on note le poids correspondant en gramme (l'ordonnée sur la Figure 2). Ainsi, plus l'animal crie tôt, pour un poids faible, plus il a mal.

Afin d'évaluer les effets du protoxyde d'azote 50% et du xénon 50% (% en volume) sur les douleurs neuropathiques induites par l'oxaliplatine, 4 groupes d'animaux, à raison de 8 rats par groupe, ont été utilisés (voir Figure 2) :
- le Groupe A ou Groupe Contrôle (triangle blanc) : les animaux ont reçu une solution de contrôle de glucose à 5% à la place de l'oxaliplatine et les administrations ont été associées à un mélange gazeux contrôle N₂/O₂ (50%-50%) pendant 1 heure ;
- le Groupe B ou Groupe Oxaliplatine + Air (losange noir): les animaux ont reçu de l'oxaliplatine (2 mg/kg) et les administrations ont été associées à un mélange gazeux contrôle N₂/O₂ (50%-50%) pendant 1 heure ;
- le Groupe C ou Groupe Oxaliplatine + N₂O (carré gris): les animaux ont reçu une solution contenant de l'oxaliplatine (2 mg/kg) et les administrations ont été associées à un mélange gazeux contenant N₂O/O₂ (50%-50%) pendant 1 heure ; et
- le Groupe D ou Groupe Oxaliplatine + xénon (rond gris): les animaux ont reçu une solution contenant de l'oxaliplatine (2 mg/kg) et les administrations ont été associées à un mélange gazeux contenant Xe/O₂ (50%-50%) pendant 1 heure.

Les Groupes A, B et C sont donnés à titre comparatif, alors que le Groupe D est selon la présente invention.

Suite aux administrations d'oxaliplatine, les seuils de douleurs du Groupe B (oxaliplatine + air) sont significativement diminués, à savoir environ 150g par rapport à 200g dans le Groupe A, révélant une hypersensibilité à la douleur ou hyperalgésie chez les rats du groupe B qui ont reçu de l'oxaliplatine. Ceci confirme que cette molécule présente l'inconvénient d'engendrer des douleurs neuropathiques.

La significativité des résultats est représentée par l'étoile proche des losanges noir sur la Figure 2.

De façon intéressante, on remarque que cette hypersensibilité à la douleur persiste bien au delà de la dernière administration d'oxaliplatine. En effet, comme on peut le voir sur la Figure 2, la dernière administration a eu lieu à J25 et les hypersensibilités à la douleur sont encore présentes plus de 2 semaines après (J42).

A l'inverse, les Groupes C et D dans lesquels les rats ont inhalé du N₂O (hors invention) ou du xénon (invention) après administration d'oxaliplatine ne présentent aucun signe d'hypersensibilité à la douleur.

Ainsi, les courbes respectives (carré gris et rond gris) pour les Groupes C et D ne sont pas différentes de la courbe du Groupe A (triangle blanc) faisant office de Groupe Contrôle.

En d'autres termes, les administrations de mélange gazeux contenant du xénon ont permis, de manière surprenante, de prévenir ou traiter les hypersensibilités à la douleur induites par les administrations successives d'oxaliplatine.

Ceci constitue une avancée notable au plan médical car, appliqué aux patients humains, ces inhalations de xénon permettraient enfin de soulager les douleurs que rencontrent les patients cancéreux subissant des chimiothérapies à base d'oxaliplatine ou d'autres substances anticancéreuses.

Par ailleurs, de façon encore plus surprenante, l'effet du xénon dure dans le temps puisque, même à la suite de la dernière administration d'oxaliplatine, l'hypersensibilité à la douleur de longue durée est totalement prévenue. En fait, ces essais montrent que l'effet préventif du xénon dure plus de 2 semaines.

Ces essais mettent bien en évidence que le xénon administré par inhalation permet de prévenir et de traiter les douleurs neuropathiques induites par les substances de chimiothérapies anticancéreuses, telle l'oxaliplatine.

Le xénon gazeux mélangé à de l'oxygène ou à de l'air par exemple, peut dès lors être mis en oeuvre pour traiter, prévenir, diminuer, minimiser... les douleurs neuropathiques qui sont des effets secondaires négatifs de l'usage de certaines substances anticancéreuses, tels les sels de platine, lesquelles peuvent être administrées seules ou sous forme de « cocktail » de plusieurs substances.

En d'autres termes, la présente invention vise aussi à englober une méthode de traitement thérapeutique dans laquelle on administre à un patient atteint d'un cancer, une substance anticancéreuse susceptible de conduire à des douleurs neuropathiques en tant qu'effets secondaires négatifs chez ledit patient, et dans laquelle on co-administre par inhalation audit patient du xénon, ou un mélange Xe/N₂O pour palier lesdites douleurs neuropathiques engendrées par ladite substance anticancéreuse, c'est-à-dire pour diminuer ou éliminer totalement lesdites douleurs et ce, de manière durable dans le temps.

## Revendications

1. Médicament inhalable gazeux contenant du xénon en tant que principe actif pour une utilisation par inhalation pour prévenir et/ou pour traiter au moins une douleur neuropathique induite par au moins une substance de chimiothérapie anticancéreuse administrée à un patient atteint d'un cancer, ladite substance de chimiothérapie anticancéreuse étant choisie parmi les sels de platine.

2. Médicament pour son utilisation selon la revendication précédente, **caractérisé en ce que** la substance de chimiothérapie anticancéreuse comprend un ou plusieurs sels de platine choisis parmi la cisplatine, l'oxaliplatine et le carboplatine.

3. Médicament pour son utilisation selon l'une des revendications précédentes, **caractérisé en ce que** la substance de chimiothérapie anticancéreuse comprend de l'oxaliplatine.

4. Médicament pour son utilisation selon l'une des revendications précédentes, **caractérisé en ce que** la proportion volumique efficace de xénon est comprise entre 5 et 70%, préférentiellement moins de 60%.

5. Médicament pour son utilisation selon l'une des revendications précédentes, **caractérisé en ce qu'**il contient en outre une proportion volumique d'oxygène (O₂) d'au moins 21%.

6. Médicament pour son utilisation selon l'une des revendications précédentes, **caractérisé en ce qu'**il contient, en outre, un gaz additionnel choisi parmi le protoxyde d'azote, l'argon, l'hélium, le néon, le krypton, le NO, le CO, le sulfure d'hydrogène (H₂S) et l'azote.

7. Médicament pour son utilisation selon l'une des revendications précédentes, **caractérisé en ce qu'**il est co-administré avec la substance de chimiothérapie anticancéreuse.

8. Médicament pour son utilisation selon l'une des revendications précédentes, **caractérisé en ce que** le patient est un être humain.

9. Médicament pour son utilisation selon l'une des revendications précédentes, **caractérisé en ce qu'**il contient une proportion volumique efficace de xénon est comprise entre 10 et 50%, préférentiellement moins de 30%.

10. Médicament pour son utilisation selon l'une des revendications précédentes, **caractérisé en ce que** le patient est atteint d'un cancer du colon ou du rectum.

11. Médicament pour son utilisation selon l'une des revendications 1 ou 4 à 9, **caractérisé en ce que** le patient est atteint d'un cancer du sein, du poumon (non à petites cellules), de l'ovaire ou de la prostate ou d'un cancer gastrique ou des voies aérodigestives supérieures.

12. Médicament pour son utilisation selon l'une des revendications précédentes, **caractérisé en ce qu'**il est administré par inhalation pendant au moins 15 minutes par jour, de préférence au moins 30 minutes par jour.

## Patentansprüche

1. Inhalierbares gasförmiges Arzneimittel, das Xenon als Wirkstoff enthält, für eine Verwendung durch Inhalation zur Vorbeugung und/oder zur Behandlung mindestens eines neuropathischen Schmerzes, der durch mindestens eine Substanz zur krebsbekämpfenden Chemotherapie, die einem an Krebs erkrankten Patienten verabreicht wird, verursacht wird, wobei die Substanz zur krebsbekämpfenden Chemotherapie aus den Platinsalzen ausgewählt ist.

2. Arzneimittel zur Verwendung nach dem vorhergehenden Anspruch, **dadurch gekennzeichnet, dass** die Substanz zur krebsbekämpfenden Chemotherapie ein oder mehrere Platinsalze enthält, die aus Cisplatin, Oxaliplatin und Carboplatin ausgewählt sind.

3. Arzneimittel zur Verwendung nach einem der vorhergehenden Ansprüche, **dadurch gekennzeichnet, dass** die Substanz zur krebsbekämpfenden Chemotherapie Oxaliplatin enthält.

4. Arzneimittel zur Verwendung nach einem der vorhergehenden Ansprüche, **dadurch gekennzeichnet, dass** das wirksame Volumenverhältnis von Xenon zwischen 5 und 70 %, vorzugsweise weniger als 60 % beträgt.

5. Arzneimittel zur Verwendung nach einem der vorhergehenden Ansprüche, **dadurch gekennzeichnet, dass** es ferner ein Volumenverhältnis von Sauerstoff (O₂) von mindestens 21 % enthält.

6. Arzneimittel zur Verwendung nach einem der vorhergehenden Ansprüche, **dadurch gekennzeichnet, dass** es ferner ein weiteres Gas enthält, das aus Distickstoffoxid, Argon, Helium, Neon, Krypton, NO, CO, Schwefelwasserstoff (H₂S) und Stickstoff ausgewählt ist.

7. Arzneimittel zur Verwendung nach einem der vorhergehenden Ansprüche, **dadurch gekennzeichnet, dass** es zusammen mit der Substanz zur krebsbekämpfenden Chemotherapie verabreicht wird.

8. Arzneimittel zur Verwendung nach einem der vorhergehenden Ansprüche, **dadurch gekennzeichnet, dass** der Patient ein Mensch ist.

9. Arzneimittel zur Verwendung nach einem der vorhergehenden Ansprüche, **dadurch gekennzeichnet, dass** es ein wirksames Volumenverhältnis von Xenon zwischen 10 und 50 %, vorzugsweise weniger als 30% enthält.

10. Arzneimittel zur Verwendung nach einem der vorhergehenden Ansprüche, **dadurch gekennzeichnet, dass** der Patient an einem Dickdarmkrebs oder Mastdarmkrebs erkrankt ist.

11. Arzneimittel zur Verwendung nach einem der Ansprüche 1 oder 4 bis 9, **dadurch gekennzeichnet, dass** der Patient an einem Brustkrebs, (nicht kleinzelligem) Lungenkrebs, Eierstockkrebs oder Prostatakrebs oder an einem Magenkrebs oder einem Krebs des oberen Aerodigestivtrakts erkrankt ist.

12. Arzneimittel zur Verwendung nach einem der vorhergehenden Ansprüche, **dadurch gekennzeichnet, dass** es durch Inhalation während eines Zeitraums von mindestens 15 Minuten am Tag, vorzugsweise mindestens 30 Minuten am Tag, verabreicht wird.

## Claims

1. Gaseous inhalable medicine containing xenon as the active ingredient for use by inhalation to prevent and/or treat at least one neuropathic pain induced by at least one anti-cancer chemotherapy substance administered to a patient suffering from cancer, said anti-cancer chemotherapy substance being selected from among platinum salts.

2. Medicine for use according to the preceding claim, **characterised in that** the anti-cancer chemotherapy substance comprises one or more platinum salts selected from among cisplatin, oxaliplatin and carboplatin.

3. Medicine for use according to either of the preceding claims, **characterised in that** the anti-cancer chemotherapy substance comprises oxaliplatin.

4. Medicine for use according to any of the preceding claims, **characterised in that** the effective volumetric proportion of xenon is between 5 and 70 %, preferably less than 60 %.

5. Medicine for use according to any of the preceding claims, **characterised in that** it also contains a volumetric proportion of oxygen (O₂) of at least 21 %.

6. Medicine for use according to any of the preceding claims, **characterised in that** it also contains an additional gas selected from among nitrous oxide, argon, helium, neon, krypton, NO, CO, hydrogen sulphide (H₂S) and nitrogen.

7. Medicine for use according to any of the preceding claims, **characterised in that** it is coadministered with the anti-cancer chemotherapy substance.

8. Medicine for use according to any of the preceding claims, **characterised in that** the patient is a human being.

9. Medicine for use according to any of the preceding claims, **characterised in that** it contains an effective volumetric proportion of xenon is between 10 and 50 %, preferably less than 30 %.

10. Medicine for use according to any of the preceding claims, **characterised in that** the patient is suffering from colon or rectal cancer.

11. Medicine for use according to any of claims 1 or 4 to 9, **characterised in that** the patient is suffering from breast cancer, (non-small-cell) lung cancer, ovarian cancer or prostate cancer or from a gastric cancer or a cancer of the upper aerodigestive tracts.

12. Medicine for use according to any of the preceding claims, **characterised in that** it is administered by inhalation for at least 15 minutes per day, preferably at least 30 minutes per day.
